(19) Europäisches Patentamt

European Patent Office

Office européen des brevets

(11) Publication number : **0 468 637 A1**

# (12) EUROPEAN PATENT APPLICATION

(21) Application number : **91305563.8**

(22) Date of filing : **19.06.91**

(51) Int. Cl.⁵ : **A61K 47/48, C12P 21/08**

(30) Priority : **22.06.90 US 542139**

(43) Date of publication of application :
**29.01.92 Bulletin 92/05**

(84) Designated Contracting States :
**AT BE CH DE DK ES FR GB GR IT LI NL SE**

(71) Applicant : **ELI LILLY AND COMPANY**
**Lilly Corporate Center**
**Indianapolis Indiana 46285 (US)**

(72) Inventor : **Johnson, David Arthur**
**11416 Lakeshore Drive West**
**Carmel, Indiana 46032 (US)**
Inventor : **Owens, Rebecca Anne**
**2013 Glenridge Drive**
**Indianapolis, Indiana 46218 (US)**

(74) Representative : **Hudson, Christopher Mark et al**
**Erl Wood Manor**
**Windlesham Surrey GU20 6PH (GB)**

(54) In vivo targeting with bifunctional antibodies.

㊗    This invention belongs to the field of immunology and provides a method for designing and constructing bispecific antibodies whose binding profiles are more desirable than those of the monospecific antibodies from which they were derived. The invention is exemplified in part by a bispecific antibody type that preferentially binds to some carcinomas and, at the same time, achieves a much decreased cross-reactivity with critical normal tissue as compared to the parent antibodies. The invention is not limited to cancer therapy, and its scope envisions the ability to selectively direct a wide variety of agents to particular sites in the body for an assortment of purposes while reducing the level of cross-reactivity to undesired locations.

EP 0 468 637 A1

This invention belongs to the field of immunological targeting and provides bispecific antibodies (BAbs), immuno-conjugates and methods of use. The BAbs achieve decreased cross-reactivity with critical normal tissue while maintaining avid target specificity. The scope of the invention envisions the ability to selectively direct a wide variety of agents to particular sites in the body for an assortment of purposes while reducing the level of cross-reactivity to undesired locations.

Targeting therapeutic and diagnostic agents to tissues, cells, and specific structures in the body has long been a goal of medical practitioners and is desirable for a variety of reasons. Physiological targeting embraces the delivery of medications to specific bodily sites while sparing healthy tissues the effects of the treatment. As diagnostics, targeting agents identify, localize, and monitor particular disease states. This type of therapy and diagnosis is already possible to differing degrees using a number of methods.

The first and most basic technique for site directed therapy is through localized administration. This rather simplistic method is very effective when targeting areas of the body that are readily accessible. Topical administration as well as intercavitary therapy are examples. Limitations arise when the desired site cannot be reached or when the agent rapidly diffuses away from the point of application.

Another approach utilizes a drug's chemical and physical properties. These pharmacokinetic properties determine how, in which organs and to what extent the body will distribute and metabolize the drug. Pharmacokinetic properties can be manipulated to favorably alter a drug's biodistribution and thereby direct it more towards certain tissues and away from others. There are also limitations to this approach because any changes made in the molecule that are designed to favorably alter the drug's biodistribution risks affecting its efficacy. Thus, a balance between potency and biodistribution is often required to achieve pharmacokinetic directed therapy and diagnosis.

Immuno-targeting requires the use of antibodies to direct and localize substances to the desired site in the body. It can be used either by directly linking the therapeutic agent to the antibody or by using the antibody as a localized artificial receptor on cells or structures of interest. The approach operates at the cellular level and requires the presence of an antigenic determinant to which antibodies can preferentially bind. After the advent of monoclonal antibodies (MAbs) in the mid seventies, there were very high expectations for immuno-site directed therapy. Many researchers believed that a host of diseases, especially cancer, might be effectively treated or cured using this so called "magic bullet". The idea of targeting MAb linked chemotherapeutics or toxins exclusively to cancerous cells appeared to be a rational means of eliminating toxic side effects inherent to chemotherapy. Numerous obstacles associated with this approach were soon confronted, and the most fundamental was the apparent lack of tumor specific antigens. In the absence of antigens unique to malignant cells, researchers have relied on tumor associated antigens (TAA) as targets for immunotherapy. TAAs are found on normal as well as tumor tissues and cause antibodies to cross-react with normal tissues. Thus, cross-reacting normal tissues decrease targeting and often limit therapeutic index.

The above discussion shows that any targeting in the body is a balance between the amount localized to the target and the amount localized to non-target sites. The more the agent is localized to the target and the less to non-targets, the better the targeting. Thus, two possibilities exist for improving targeting. They are to increase the amount localized at the desired site while maintaining or decreasing the amount localized elsewhere, or to maintain or increase the amount localized at the desired site while decreasing the amount localized elsewhere. This invention does the latter by providing BAbs that maintain avid target specificity while diminishing unwanted cross-reactivity with certain critical tissues. The invention encompasses unique types of BAbs, immuno-conjugates and methods of use for therapy and diagnosis.

The invention is a bispecific antibody constructed of two different monospecific antibodies which have binding specificities to two different, adjacent, target associated epitopes situated on the surface of the target structure but which are not adjacently co-expressed on the surface of non-target structures, which BAb has binding avidity to non-target structures which is less than that of either monospecific antibody. The invention includes a BAb reactive with the L/1C2 antigen and the KS1/4 antigen as well as a BAb reactive with the L/1C2 antigen and another tumor associated antigen that is found adjacent to the L/1C2 antigen on tumor cells but is not significantly co-expressed on the surface of normal tissue. The invention also encompasses a BAb reactive with the KS1/4 antigen and another tumor associated antigen that is found adjacent to the KS1/4 antigen on tumor cells but is not significantly coexpressed on the surface of normal tissue. The invention further contains immuno-conjugates constructed using said BAbs as well as therapeutic and diagnostic methods of use for such immuno-conjugates.

Also included in the invention is a method of selectivity directing a beneficial substance to a target structure in an animal which comprises administering to that animal a bispecific antibody or immuno-conjugate as herein described. A further aspect of the invention is a process for preparing a bispecific antibody or immuno-conjugate, which comprises identifying suitable antibodies, preparing monovalent species of said antibodies, joining dissimilar monovalent species, and optionally reacting said bispecific antibody with a molecular or atomic moiety to form an immuno-conjugate.

Because the use of immunological terms in the scientific literature is often imprecise, a list of definitions as used in this document follows.

bispecific antibody (BAb) - bifunctional antibody; an antibody, antibody fragment (including but not limited to F(ab')$_2$ fragments) or bivalent binding molecule capable of simultaneously binding two different and distinct types of antigens or epitopes which is constructed by either chemical or cell fusion techniques.

monospecific antibody - an antibody or antibody fragment capable of binding only one distinct type of antigen or epitope irrespective of valency.

binding specificity - a term used to refer to the particular epitope to which an antibody binds.

epitope - the particular molecular configuration to which an antibody combining site binds.

adjacent - sufficiently close to allow for simultaneous bivalent binding by a bispecific antibody or fragment.

target structure - a bodily site ranging from an aggregate of macromolecules to a group of dispersed similar cells to entire organs or organ systems at which a substance is directed.

immuno-conjugate - a molecule formed by linking in any fashion an antibody with a molecular or atomic moiety while retaining the binding specificity of the antibody.

avidity - a measure of an antibody's rate or firmness of combination with an antigen.

monovalent binding - a single combining interaction between an antibody and an antigen.

bivalent antibody - an antibody having two antigen combining sites irrespective of specificity.

bivalent binding - two combining interactions between an antibody and an antigen irrespective of specificity.

selectively directing - the ability to localize a substance to a desired site in the body; includes single and multistep methods.

critical tissue - bodily tissue that presents cross-reactive antigens to immuno-conjugates and cause a limitation to immuno-targeting.

parent antibody - an antibody or fragment used in the construction of a BAb and which comprises one binding arm of the BAb.

The BAbs of the present invention are useful as targeting vehicles capable of delivering a wide variety of beneficial agents for numerous purposes to pre-selected sites in the body. In the past decade, an entire field has emerged around immuno-targeting technology as evidenced by the scientific journal Antibody Immuno-conjugates, and Radiopharmaceuticals (ed. Order, S.) Mary Ann Liebert, Inc., New York. Also the following review articles will help establish the state of the art with respect to immuno-conjugates. They are Ghose et al., CRC Crit. Rev. in Therapeutic Drug Carrier Systems 3, 263-359 (1987); Blare and Ghose, J. Immunol. Methods 59, 129-143 (1983); and Ghose et al., Methods in Enzymol. 93, 280-333 (1983). Issued U.S. patents such as 4,263,279, Pharmacologically Active Compositions Containing Adriamycin and Daunomycin, Sela et al.; 4,671,958, Antibody Conjugates for the Delivery of Compounds to Target Sites, Rodwell and McKearn; 4,801,688, Hydrazone Immunoglobulin Conjugates, Laguzza and Nichols; and 4,845,200, Immunoglobulin Conjugates, Cullinan et al. further demonstrate the well established state of the art in this field as does EPO publication number 0 354 728 A2, Cytotoxic Drug Conjugates, Cullinan et al.; EP 0 354 729 A2, Cytotoxic Drug Conjugates, Johnson et al.; and EP 0 368 668 A2, Antibody-drug Conjugates, Barton. Therefore, the ordinarily skilled artisan will readily know what type of beneficial agent may be coupled to a BAb of the invention to attain a certain targeting goal. He will also know how to couple the agent to a BAb and how best to administer the resulting immuno-conjugate to achieve the desired results.

The avidity of an antibody-antigen interaction is affected by the number of physical bonds between the two molecules. The avidity constant of an antibody bound to two epitopes is greater than that of the same antibody bound to one epitope because of the additional interaction. In a dynamic system such as the body where thermodynamic properties result in dislodging weakly bound molecules, the difference between monovalent and bivalent binding can manifest itself as greatly reduced cross-reactivity with the tissue or cells where only monovalent binding is possible. This invention exploits a monospecific antibody whose utility as a targeting agent is diminished due to its cross-reactive nature by using another such antibody to prepare a BAb with binding properties superior to either parent antibody.

For the invention to function properly, the BAb must demonstrate bivalent binding at the target and monovalent binding at cross-reactive tissues. These binding characteristics are achieved when the chosen epitopes are located adjacent to each other on the target structure and are not adjacently co-expressed on critical cross-reactive tissues. Like the parent antibodies, a BAb directed at such an antigen or epitope pair will bind the target structure in a bivalent manner. However, unlike the parent antibodies, the BAb will monovalently bind any cross-reactive tissues. The less avid monovalent interaction will dissociate faster than the bivalent interaction and in time leave the majority of BAb bound to the target. Thus, by employing the BAb, better targeting is achieved because the level of cross-reactivity inherent to either parent antibody is reduced while leaving the more avid bivalent binding at the target unaffected.

In order to obtain a BAb with superior binding properties, the choice of which antigen or epitope pair the BAb should bind is crucial. This decision is the foundation of the invention and will ultimately dictate the invention's utility. Therefore, a careful examination of the antigenic diversity expressed on the target substance is strongly advised.

With this invention in mind, the antigenic make-up of a target structure is best explored using antibodies because the tools used in probing the target can later be exploited to construct the invention. If some other means is employed, antibodies directed toward the molecules of interest will later have to be acquired or developed in order to make and use the invention. The antigenic make-up of the target can be probed using commercially available antibodies or by using antibodies that have been raised in response to the target. Immunologists of ordinary skill are entirely able to produce antibodies through well established techniques or to choose appropriate antibodies from sources such as the catalogue of the American Type Culture Collection (Rockville MD) and Linscott's Directory of Immunological and Biological Reagents, published by Linscott's Directory, 40 Glen Drive, Mill Valley, CA., 94941.

The following is a non-exhaustive list of commercially available MAbs that might be used for purposes of this invention.

| Antigen | Type | Designation | Source |
|---|---|---|---|
| Human amniotic mesenchyme | $IgG_1$ | GB10 | Accurate Chem. & Sci. Corp., Westbury, NY |
| Human B cells | $IgG2_a$ | OKB7 | Ortho-Mune, Raritan, NJ |
| Human Bladder Urothelium | $IgG_1$ | Om5 | Cambridge Research Lab, Cambridge, MA |
| Human breast carcinoma | $IgG2_a$ | HuTu.ml | BSI, Indianapolis, IN |
| Human carcinoma | $IgG_3$ | KC4 | Coulter Immunology, Hialeah, FL |
| Human granulocytes | $IgG_1$ | FMC11 | BSI, Indianapolis, IN |
| Human keratinocytes | $IgG_3$ | BL9 | Accurate Chem. & Sci. Corp., Westbury, NY |
| Human neutrophils | $IgG_1$ | PMN13F6 | Accurate Chem. & Sci. Corp., Westbury, NY |
| Human platlets | $IgG_1$ | 57.2 | Amac Inc, Westbrook, ME |
| Human T cells | $IgG_1$ | T4 | Coulter Immunology, Hialeah, FL |
| Sheep RBCs | $IgG_1$ | Sp3HL | Accurate Chem. & Sci. Corp., Westbury, NY |
| Human alpha-fetoprotein | $IgG_1$ | AFP26 | BSI, Indianapolis, IN |
| Human alpha-1-antitrypsin | $IgG2_a$ | 1101 | Biodesign Inc., Kennebunkport, ME |

| | | | |
|---|---|---|---|
| Human alpha-2-macroglobulin | IgG2$_b$ | 1204 | "          "          " |
| Human acetylcholine receptor | IgG2$_b$ | MOAV/AR4 | Accurate Chem. & Sci. Corp., Westbury, NY |
| Human actin | IgG2$_a$ | 1A4 | "          "          " |
| Basement membrane | IgG$_1$ | A3211 | Chemicon Int'l. El Segundo, CA |
| Blood group antigen A | IgG$_3$ | T36 | Cambridge Research Lab, Cambridge, MA |
| Blood group antigen B | IgG$_1$ | 23-48 | Wistar Institute, Philadelphia, PA |
| Blood group antigen H | IgG$_3$ | A7-206 | Cambridge Research Lab, Cambridge, MA |
| Blood group antigen Le$^a$ | IgG$_1$ | T174 | Cambridge Research Lab, Cambridge, MA |
| Blood group antigen Le$^b$ | IgG$_3$ | CO29.4 | Wistar Institute, Philadelphia, PA |
| Breast carcinoma ag. (BCA 225) | IgG$_1$ | CU18 | Cambridge Research Lab, Cambridge, MA |
| Human C3b receptor CR1 | IgG$_1$ | J3D3 | Amac Inc, Westbrook, ME |
| Human C3bi receptor CR3 | IgG$_1$ | 2LPM19c | Accurate Chem. & Sci. Corp., Westbury, NY |
| Human C5a | IgG2$_b$ | MAK557 | Calbiochem, San Diego, CA |
| Human calmodulin | IgG$_1$ | - | Chemicon Int'l. El Segundo, CA |

| | | | |
|---|---|---|---|
| Human carcino-embryonic antigen | IgG$_1$ | FO23C5 | Accurate Chem. & Sci. Corp., Westbury, NY |
| Human chorionic gonadotropin (hCG) | IgG$_1$ | W14 | "            "            " |
| Alpha hCG | IgG$_1$ | 651 | Amac Inc, Westbrook, ME |
| Beta hCG | IgG$_1$ | PZ15 | Zymed Laboratories, San Francisco, CA |
| Epidermal growth factor receptor | IgG2$_a$ | 425 | Wistar Institute, Philadelphia, PA |
| Human ferritin | IgG2$_a$ | 501 | Biodesign, Inc. Kennebunkport, ME |
| Human Fibronectin | IgG$_1$ | IST-1&2 | Accurate Chem. & Sci. Corp., Westbury, NY |
| Gangliosides GD2 & GD3 | IgG$_3$ | NE36.1 | Wistar Institute, Philadelphia, PA |
| Human Histones | IgG2 | - | Chemicon Int'l. El Segundo, CA |
| Human insulin receptor | IgG2$_a$ | B6 | Amac Inc, Westbrook, ME |
| Human interlukin 2 receptor | IgG2$_a$ | IL-2R1 | Coulter Immunology, Hialeah, FL |
| Melanoma antigen GD3 | IgG$_3$ | R24 | Cambridge Research Lab, Cambridge, MA |
| Nerve growth factor receptor | IgG$_1$ | 20.4 | Wistar Institute, Philadelphia, PA |
| Neuronal cell surface antigen | IgG2$_a$ | - | Chemicon Int'l. El Segundo, CA |
| Oncogene c-myc product | IgG$_1$ | CT14 | Cambridge Research, Valley Stream, NY |
| Human transferrin receptor | IgG$_1$ | L12.2E6.1 | Accurate Chem. & Sci. Corp., Westbury, NY |
| Tubulin | IgG rat | YL1-2 | "            "            " |
| Human tumor assoc. glycoprotein | IgG$_1$ | B72.3 | Biomedical Technologies Stoughton, MA |

Raising antibodies against a particular target structure is another way to obtain antibodies that can be used first to probe the target's surface and later to construct the invention. The classical method of immunizing animals, drawing blood, and purifying antibodies from the serum is a possibility; however, this approach will cause difficulties when constructing a BAb because even affinity purified antibodies obtained in this manner will be heterogeneous populations of antibodies.

The method of somatic cell fusion for the generation of MAbs was first described by Kohler and Milstein in 1975. This powerful and oft practiced technique is well known to those of ordinary skill in the art and provides a more rational approach to obtain antibodies that are useful in exploring the antigenic composition of a target structure. Monoclonal antibodies are well suited for this task because one cell fusion experiment can lead to hundreds of antibodies reactive with different epitopes on the substance of interest. Usually the panel of antibodies generated will include a variety of MAb isotypes directed against the same epitope. This is particularly advantageous because some isotypes are better suited to the enzymatic reactions used in the chemical construction of BAbs. Also, when constructing BAbs through biological means, it is advantageous to use matching isotypes to better facilitate antibody assembly in the polydoma. Once a hybridoma of interest has been identified and cloned, a consistent and almost limitless source of the MAb is available. Finally, having the hybridomas that secrete the parent MAbs will allow for the bio-construction of the BAb by fusing the two different hybridoma types.

Turning again to choosing an antigen or epitope pair on the target, it is best to select dominant, highly expressed antigens. In so doing, there is a greater chance of each being adjacently co-expressed as required by the invention. High copy number on the surface of the target structure is by no means a requirement; rather it merely increases the likelihood of finding an antigen or epitope pair adjacently co-expressed on the target structure. High antigen copy number on the target structure is also naturally advantageous for targeting purposes because more binding sites are available to the targeting agent. Conversely, any cross-reacting tissue preferably should have relatively low levels of the antigen in order to minimize the level of non-target binding and to decrease the likelihood of an antigen pair being adjacently co-expressed.

Having found antibody types reactive with target antigens, one must then look to the pattern of cross-reactivity associated with the antibodies. Epitopes of interest will invariably be found on tissues or cells apart from the target and are responsible for the unwanted cross-reactivity. If no cross-reactivity were observed, the need for this invention would be averted because the problem that the invention was designed to solve would not exist. There are numerous methods for determining an antibody's binding profile, and each method has inherent advantages and disadvantages.

Immunohistology is the most practical method for determining the location and distribution of an antigen in bodily tissues. This classic technique has a long history in medical science and is well known to those of ordinary skill in the art.

Quantitative immunoassays such as Radio-Immuno-Assays (RIA) or Enzyme Linked Immuno-Sorbant Assays (ELISA) are also well suited for studying antigenic composition. These popular, commonly used assays are well known to those of ordinary skill in immunology and are rapid, quantitative, and lend themselves well to routine mass screening.

Imaging studies can be used to determine the binding profile of an antibody. This newer technique is considerably more involved than immunohistology or other solid phase assays because the imaging agent is labelled with a radionuclide, administered intravenously, and observed and recorded using specialized instruments. Radio-labelled MAbs are typically used as imaging agents due to their binding fidelity and the great variety of available specificities. Imaging studies provide several advantages over conventional immunohistology and other solid phase assays. Over time, researchers can follow the imaging agent as it progresses through the body. How the agent disperses through the body, where it localizes, how long it remains at a site, and through what route it is excreted are just some of the parameters that can be measured in one imaging study. Despite being a relatively new technique, many imaging studies have been described in the scientific literature and are well within the grasp of the skilled artisan.

Once an antigen or epitope pair that is co-expressed on the target structure but is not co-expressed on non-target tissues has been selected, it must be determined if they are adjacently co-expressed on the target structure. For purposes of this invention, adjacently co-expressed is functionally defined as sufficiently close to allow for simultaneous bivalent binding by the BAb. Therefore, the BAb must be constructed to test for this requirement. If the constructed BAb meets this last requirement, the basic functional element of the invention is complete.

There are two basic approaches to constructing BAbs, and both are well known to those of ordinary skill in their respective arts. Chemical synthesis begins with purified amounts of two distinct antibody species and proceeds by dissociating the two heavy and light chain pairs into two half molecules. It culminates when two dissimilar half molecules are covalently joined. Biological construction is accomplished by fusing together two different antibody secreting cells. The method is identical to the technique used to produce MAbs except that in BAb construction, each cell type to be fused secretes different antibody specificities.

Chemical synthesis considerably predates any biological means of constructing BAbs. Nisonoff et al., Nature, 194: 355 (1962), describes a method for enzymatically cleaving off the $F_c$ portion of a rabbit IgG molecule using pepsin. The remaining disulfide bond linking together the two heavy chains was selectively reduced yielding two half molecules. After repeating these steps for a different IgG, two dissimilar half molecules were oxidatively combined to form a BAb. More recent work detailing novel chemical syntheses for BAbs is found in PCT application, WO 85/04811, and EPO publication number, O 241 907 A2. Viewing the past quarter century of work in this field, it is evident that the chemical construction of BAbs is well known to those of ordinary skill in the art.

The more recent bio-construction of BAbs has been disclosed in U.S. Patent 4,474,893 and is incorporated by reference. The patent describes methods for producing and selecting polydomas derived by fusing two hybridomas or a hybridoma and a spleen cell from an immunized donor. Milstein and Cuello, Nature, 305: 537 (1983), report similar methods. Chervonsky et al., Molecular Immunology, 29, 9: 913 (1988), also reports the bio-construction of BAbs and describes a new selection method. All polydomas secrete a mixture of antibodies consisting of a BAb with predefined specificities as well as the parent MAb forms and forms composed of mixtures of the parent heavy and light chains. Unlike most chemically derived BAbs, all bio-constructed antibodies are fully formed and include the $F_c$ region. The presence or absence of the $F_c$ region is an important factor to consider since it is the complement binding domain of antibodies and also contributes to a BAb's immunogenic properties.

In a preferred embodiment, the invention is used to target cancerous tissue and to minimize cross-reactivity with normal critical tissues such as colon, kidney, red blood cells and endothelial cells in humans. Antigens consistent with this embodiment include TAAs such as carcino-embryonic antigen (CEA), epidermal growth factor receptor (EGFr), transferrin receptor (Tr), human milk fat globule membrane antigen, beta human chorionic gonadotropin, 17-1A, KS1/4, L/1C2, and tumor-associated glycoprotein 72 (TAG-72). In a more preferred embodiment, one arm of the BAb will bind the L/1C2 antigen and the other arm will bind one of the above TAAs, or one arm will bind the KS1/4 antigen and the other arm will bind one of the above TAAs. A more highly preferred embodiment will simultaneously bind the L/1C2 and KS1/4 antigens and will be less cross-reactive with red blood cells, endothelial cells and normal colon tissue.

The following examples describe how one embodiment of the invention was constructed and will help illustrate the invention. While the following detailed examples focus on targeting tumor tissues, other tissues, cellular, and non-cellular target structures of interest are within the scope of the invention. The following descriptions are merely used to illustrate how a BAb, whose specificities are carefully chosen, can more effectively target a site in the body by avoiding problematic cross-reactivity.

All of the following examples were performed using two different murine $IgG_1$ MAb types. L2-KS binds to a 40-kDa glycoprotein human TAA called KS1/4. The L2-KS hybriboma is on deposit with the American Type Culture Collection and has been assigned the ATCC designation HB 9940. L/1C2 reacts with an approximately 110-140 kDa glycoprotein human TAA found primarily on squamous carcinomas and adenocarcinomas. The L/1C2 hybridoma is on deposit with the ATCC under accession number HB 9682. Both hybridoma cultures were deposited in accordance with the Budapest Treaty.

EXAMPLE 1

Selection of Parental MAbs for Construction of BAb L/1C2 - L2-KS

A. Staining of frozen sections:

Tissue and tumor antigenic distribution studies were carried out using a modified avidin-biotin technique. Using a cryostat microtome, frozen samples of human biopsy or autopsy tissues were cut into 4-6 μm sections and placed onto gelatin coated slides. The sections were then air dried and fixed in acetone for five minutes. Following preincubation with 5% agamma-globulin horse serum in PBS (GIBCO, Grand Island, NY), the sections were sequentially incubated with L/1C2, followed by biotinylated horse anti-mouse immunoglobulin (Vectorlabs, Burlingame CA) and, lastly, a complex of avidin and horseradish peroxidase. The samples were washed between steps in PBS, pH 7.2 - 7.4, containing 1% bovine serum albumin. Color was developed using 3,3′-diaminobenzidine and sections were counterstained with hemotoxylin.

B. Red blood cell reactivity:

Red blood cell reactivity was determined using an agglutination assay. Fresh blood was drawn from volunteers into heparinized syringes and centrifuged. The plasma and the buffy coat of white cells were aspirated off, leaving red blood cells which were then washed three times in PBS. A 2% (weight/volume) suspension of red blood cells to PBS was prepared, and 0.1 ml of the suspension was mixed with 0.1 ml of MAb (10 μg/ml). The mixture was incubated for 15 minutes at room temperature, then centrifuged for 15 seconds at 400 X G. Light microscope slides of the red blood cell pellet were prepared and read for agglutination.

C. Results:

Table 1 is a summary list of the critical differences in normal tissue expression of the L/1C2 and the KS1/4 antigens.

## TABLE 1

| Tissue Site | L/1C2 | KS1/4 |
|---|---|---|
| Colon epithelium | Weak to negative | Strong positive |
| Blood Vessel endothelium | Positive | Negative |
| Duodenum | Moderate positive | Strong positive |
| Skin squamous epithelium | Positive | Negative |
| Ovary | Negative | Positive |
| Red blood cells | Positive | Negative |

Both L/1C2 and KS1/4 reactive MAbs have good reactivity with certain adenocarcinomas and certain squamous carcinomas. Tumor cell lines derived from such tumors often co-express the antigens with which these antibodies react. These tumor reactivity data therefore suggest that these two antibodies may be candidates for construction of a BAb.

A second critical requirement is that the two antibodies differ with respect to the expression of their respective antigens on critical normal tissue. Table 1 summarizes these critical differences.

L/1C2 has the disadvantage of being reactive with red blood cells, blood vessel endothelium and normal squamous epithelium. Reduction of these reactivities relative to tumor reactivity is desirable due to the significant antigenic sink which these sites represent. Also, localization of high concentrations of toxic agents to endothelial cells is clearly not desirable. In contrast, the KS1/4 antigen has the advantage of having no reactivity with red blood cells, blood vessel endothelium, and normal squamous epithelium.

Table 1 also reveals that KS1/4 has the disadvantage of being strongly reactive with colonic epithelium, duodenal epithelium, and ovary. Again these tissues represent significant antigenic sinks to which localization of antibody or toxic agents is not desirable. In particular, it has been reported by Schneck et al., Fourth International Conference of Monoclonal Antibody Immunoconjugates for Cancer, San Diego, CA, March 31, 1989 that KS1/4 Mab linked to a toxic vinca derivative results in a dose limiting toxicity when localized to human duodenum. L/1C2 in contrast has only weak to moderate reactivity with colonic epithelium and duodenal epithelium with no reactivity towards ovary.

A BAb composed of an L/1C2 an and an L2-KS arm will have the advantage of only monovalent (less avid) binding with red blood cells, blood vessel endothelium, normal squamous epithelium, and ovary. Thus, reduced binding with colonic and duodenal epithelia will occur. In contrast, the adjacent expression of the L/1C2 and the KS1/4 antigens on tumor target sites will lead to retention of bivalent (more avid) binding at those sites. The overall effect will be increased tumor binding relative to normal tissue binding.

EXAMPLE 2

Chemical Construction of BAb L/1C2 - L2-KS

A. Materials:

| | |
|---|---|
| TEA: | 0.05 $\underline{M}$ triethanolamine, pH 8.0. |
| borate buffer: | 0.05 $\underline{M}$ sodium borate, 0.001 $\underline{M}$ EDTA (ethylenediaminetetraacetic acid), 0.1 $\underline{M}$ NaCl, pH 8.2. |
| cysteine: | 0.1 $\underline{M}$ cysteine free base in TEA. |

NEM: 1.0 $\underline{M}$ N-ethylmaleimide in DMF (N,N-dimethylformamide) prepared fresh.
ammonium citrate buffer: 0.05 $\underline{M}$ ammonium citrate, 0.1 $\underline{M}$ NaCl, 0.001 EDTA, pH 6.5.
DTNB: 5,5′-dithiobis(2-nitrobenzoic acid) solid.

B. Method:

L/1C2 and L2-KS F(ab′)$_2$ fragments were prepared by conventional pepsin cleavage. The F(ab′)$_2$ fragments were solvent exchanged into borate buffer using a G25 (16/10) column (Pharmacia, Piscataway, NJ). The concentrations of the antibody fragment solutions were 3.0 mg/ml for L/1C2 and 3.2 mg/ml for L2/KS.

L/1C2 F(ab′)$_2$ antibody fragments were reduced by adding 275 μls of cysteine stock solution to 2.5 mls of the fragment solution and incubating at room temperature for 1 hour. Then, to the reduced F(ab′)$_2$ fragment solution, approximately 60 mg of DTNB was added slowly with light agitation while carefully monitoring and maintaining the pH between 7 and 9 by the dropwise addition of 5 N NaOH. The pH of the solution was adjusted to 8.0 after all of the DTNB was added. The reaction continued at room temperature for 30 minutes after which the reaction was solvent exchanged into citrate buffer using a G25 column.

L2-KS F(ab′)$_2$ antibody fragments were reduced by adding 275 μls of cysteine stock solution to 2.5 mls of the fragment solution and incubating at room temperature for 2 hours. The solution was then solvent exchanged into citrate buffer using a G25 column.

To combine the two types of Fab′ fragments produced above, 1.2 moles of L/1C2 Fab′-TNB was mixed with 1.2 moles of L2-KS Fab′. The combination reaction was carried out at 4°C overnight with no agitation. NEM was then added to a 0.1 $\underline{M}$ final concentration to stop the reaction. Ammonium sulfate was then directly added to the solution to a final concentration of 0.8 $\underline{M}$. The F(ab′)$_2$ heterodimer (BAb) was purified over a phenyl sepharose FF column (Pharmacia, Piscataway, NJ) using a linear decreasing gradient of ammonium sulfate (1 $\underline{M}$ in 0.1 $\underline{M}$ phosphate, pH 7 versus 0.1 $\underline{M}$ phosphate, pH 7) from 1.0-0.4 $\underline{M}$ over two column volumes then a linear decrease from 0.4-0.1 $\underline{M}$ over 24 column volumes. The BAb eluted at approximately 0.3 $\underline{M}$ ammonium sulphate.

EXAMPLE 3

Determination of Adjacent Co-expression of L/1C2 and KS1/4 Antigens on Target Cells

A. Preparation of cells for flow cytometric competitive inhibition assay:

The human squamous carcinoma cell line T222 described by Masui et al., Cancer, 44(3):1002-7, 1984 expresses both L/1C2 and KS1/4 antigens on its surface. The line was grown in tissue culture according to standard techniques, and the cells were harvested from their growth flasks using trypsin/EDTA (GIBCO) warmed to 37°C. The single cell suspension was washed three times in cold Dulbecco's Modified Eagle's medium + 10% fetal calf serum + gentamycin (GIBCO). A sufficient number of test tubes containing aliquots of approximately $1 \times 10^6$ cells/tube were prepared and kept on melting ice.

B. Competitive inhibition:

Serial dilutions of the test BAb (L/1C2 - L2-KS) and control MAbs (L/1C2 F(ab′)$_2$, L/1C2 Fab′, L2-KS F(ab′)$_2$, L2-KS Fab′) were prepared in HBSS (Hank's Balanced Salt Solution + 0.1% NaN$_3$; GIBCO) starting at 200 μg/ml down to 3.125 μg/ml. The labelled competing antibody used in this assay was L2-KS-fluorescein isothiocyanate (FITC) which was prepared to a concentration of 10 μg/ml in HBSS. All of the above solutions were kept on melting ice protected from light.

The T222 cell-containing test tubes were spun and the supernatant was removed by aspiration leaving a well drained cell pellet. Then 50 μl of a diluted antibody sample was simultaneously added with 50 μl of the competing FITC-labelled MAb to a test tube containing a cell pellet. The tubes were vortexed and incubated on melting ice protected from light for 60 minutes. The cells were then washed one time with approximately 3 mls of HBSS after which the cells were resuspended and fixed in 1 ml of PBS + 0.5% paraformaldehyde. At this point the cell samples were ready for cell-sorting analysis and were stored in a refrigerator for about an hour until they were run on the flow cytometer.

## C. Flow cytometry:

Flow cytometric cell sorting was done with an Epics C (Coulter Epics Division, Hialeah, FL.) flow cytometer-cell sorter (FCCS). The antibody-treated T222 cells were excited with 600 MW of 488 nm light emitted from an argon ion laser. The resultant cell-associated fluorescence light was captured through 488 nm interference and 515 nm long pass absorbance optical filters. The optical alignment of the FCCS instrument was optimized and the fluorescence readings obtained were standardized using uniform fluorospheres. Measurements of log integrated green fluorescence intensity were gated on forward angle light scatter and were linearized, channel by channel, using a commercial software package called "LogMean" (Peanut Jelly Software Co., Denver CO.).

## D. Results:

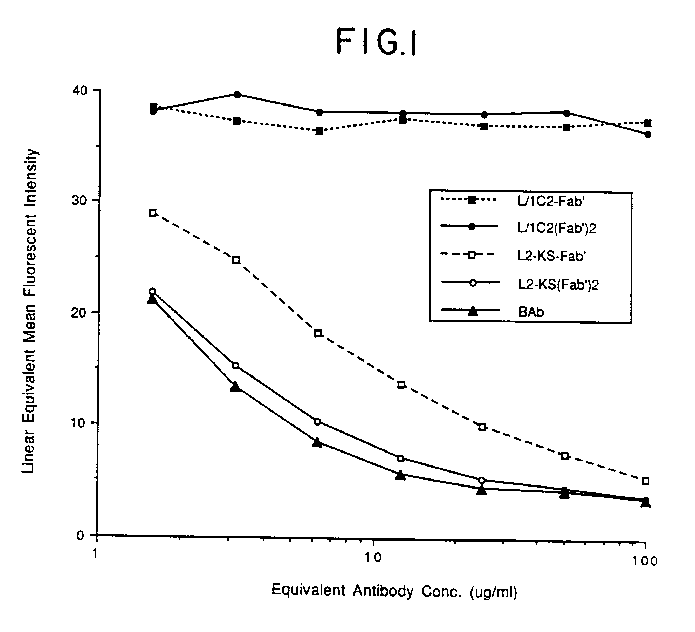

The results of the competitive inhibition assay are plotted in figure 1 and show three distinct curve types. The L/1C2-Fab' and L/1C2 F(ab')$_2$ lines run horizontally at the top of the graph. As the concentration of the labelled MAb increases relative to the unlabelled antibody, the fluorescent signal remains constant proving that L/1C2 and labelled L2-KS do not compete for the same epitope.

The next lower line demonstrates that the labelled MAb competes with the monovalent L2-KS-Fab' antibody for the same epitope. As the concentration of the unlabelled antibody fragment increases relative to the labelled MAb, the signal strength increases because the labelled MAb is able to outcompete the fragment based on its bivalent binding capabilities. The divalent labelled MAb is more avidly bound because it can remain bound to the antigen with one combining arm while the other is temporarily displaced by the fragment. The displaced arm will in turn readily displace the fragment because the other arm, which has remained bound to an adjacent epitope, has kept the displaced arm in close proximity to its epitope.

The bottom two curves are a result of the bivalent L2-KS F(ab')$_2$ or the BAb's increased avidity and hence increased ability to compete with the labelled MAb. As discussed above, bivalent antibodies bind their antigens more avidly because two interactions exist between the antibody and antigen. This is true regardless of the specificity of the combining site so long as the antibody can simultaneously bind two epitopes. When the bivalent L2-KS F(ab')$_2$ curve is compared with the monovalent L2-KS Fab' curve, the effect of the additional binding site is evidenced by the downward shift of the L2-KS F(ab')$_2$ curve. The BAb is at least as difficult, if not more difficult, to displace as is the monospecific bivalent antibody, proving that the BAb can simultaneously bind both of its epitopes. Thus, the L/1C2 and the KS1/4 antigens are adjacently coexpressed as defined in this specification.

## Claims

1. A bispecific antibody constructed of two different monospecific antibodies which have binding specificities to two different, adjacent, target structure associated epitopes situated on the surface of the target structure but which are not adjacently co-expressed on the surface of non-target structures, which bispecific antibody has binding avidity to non-target structures which is less than that inherent in either of the monospecific antibodies.

2. The bispecific antibody of Claim 1 wherein the target structure is a tumor cell.

3. The bispecific antibody of Claim 2 wherein said bispecific antibody is reactive with the L/1C2 antigen and the KS1/4 antigen.

4. The bispecific antibody of Claim 2 wherein said bispecific antibody is reactive with the L/1C2 antigen and another tumor associated antigen that is found adjacent to the L/1C2 antigen on tumor cells but is not significantly co-expressed on the surface of normal tissue.

5. The bispecific antibody of Claim 2 wherein said bispecific antibody is reactive with the KS1/4 antigen and another tumor associated antigen that is found adjacent to the KS1/4 antigen on tumor cells but is not significantly co-expressed on the surface of normal tissue.

6. An immuno-conjugate of the antibody of any one of Claims 1 to 5.

7. A pharmaceutical formulation comprising as an active ingredient a bispecific antibody or immuno-conjugate as claimed in any one of Claims 1 to 6, associated with one or more pharmaceutically acceptable carriers, excipients or diluents therefor.

8. A process for preparing a bispecific antibody or immuno-conjugate as claimed in any one of Claims 1 to 6, which comprises identifying suitable antibodies, preparing monovalent species of said antibodies, joining dissimilar monovalent species, and optionally reacting said bispecific antibody with a molecular or atomic moiety to form an immuno-conjugate.

**Claims for the following Contracting States : ES**

1. A process for preparing a bispecific antibody or immuno-conjugate constructed of two different monospecific antibodies which have binding specificities to two different, adjacent, target structure associated epitopes situated on the surface of the target structure but which are not adjacently co-expressed on the surface of non-target structures, which bispecific antibody has binding avidity to non-target structures which is less than that inherent in either of the monospecific antibodies, which comprises identifying suitable antibodies, preparing monovalent species of said antibodies, joining dissimilar monovalent species, and optionally reacting said bispecific antibody with a molecular or atomic moiety to form an immuno-conjugate.

2. A process according to Claim 1 for preparing KS1/4-L/1C2 bispecific antibody.

3. A process according to Claim 1 for preparing a KS1/4-L/1C2 immuno-conjugate.

4. A process for preparing a pharmaceutical formulation which comprises admixing a bispecific antibody or immuno-conjugate with one or more pharmaceutically-acceptable carriers, diluents of excipients therefor.

# FIG.1

Legend:
- L/1C2-Fab'
- L/1C2(Fab')2
- L2-KS-Fab'
- L2-KS(Fab')2
- BAb

Y-axis: Linear Equivalent Mean Fluorescent Intensity

X-axis: Equivalent Antibody Conc. (ug/ml)

**European Patent Office**

**EUROPEAN SEARCH REPORT**

Application Number

EP 91 30 5563

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 331 034 (NEORX CORP.) * Page 4, lines 28-37; page 6, lines 45-50; examples 1,2 * | 1,2,5-8 | A 61 K 47/48 C 12 P 21/08 |
| Y | | 1-8 | |
| Y | EP-A-0 338 846 (ELI LILLY AND CO.) * Page 5, lines 57-65; page 7, lines 47-52; claims * | 1-8 | |
| D,A | EP-A-0 368 668 (ELI LILLY AND CO.) * Examples 1,4; page 33, lines 4-20 * | 1-8 | |
| D,A | EP-A-0 354 729 (ELI LILLY AND CO.) * Examples 1,6 * | 1-8 | |
| A | WO-A-8 911 863 (GLENNIE) * Claims * | 1-8 | |

TECHNICAL FIELDS
SEARCHED (Int. Cl.5)

A 61 K
C 07 K

The present search report has been drawn up for all claims

| Place of search | Date of completion of the search | Examiner |
|---|---|---|
| THE HAGUE | 28-10-1991 | SITCH W.D.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)